# EUROPÄISCHE PATENTSCHRIFT

(11) **EP 0 869 173 B1**
(45) Veröffentlichungstag und Bekanntmachung des Hinweises auf die Patenterteilung: **12.04.2006**
(21) Anmeldenummer: 98101337.8
(22) Anmeldetag: 27.01.1998
(51) Int. Cl.: C12N 5/00, A61L 27/00

(54) **Träger für Zellkulturen**
Carrier for cell cultures
Support pour cultures cellulaires

(30) Priorität: 27.02.1997 DE 19707910
(43) Veröffentlichungstag der Anmeldung: 07.10.1998
(73) Patentinhaber: CellMed AG, 63755 Alzenau (DE)
(72) Erfinder: CellMed AG, 63755 Alzenau (DE)
(74) Vertreter: Pöhner, Wilfried Anton, Dr.

(56) Entgegenhaltungen:
- KLÖCK G. ET AL: "Biocompatibility of mannuronic acid-rich alginates" BIOMATERIALS, Bd. 18, Nr. 10, 1997, Seiten 707-713, XP004063784
- MIDDELKOOP, E. ET AL.: "Adherence, proliferation and collagen turnover by human fibroblasts seeded into different types of collagen sponges" CELL TISSUE RES, Bd. 280, 1995, Seiten 447-453, XP000996431
- SMIDSROD, O. ET AL.: "Alginates as immobilization matrix for cells" TRENDS IN BIOTECHNOLOGY, Bd. 8, 1990, Seiten 71-77, XP000095653
- HAUSELMANN, H.J. ET AL.: "Adult human chondrocytes cultured in alginate form a matrix similar to native human articular cartilage" THE AMERICAN PHYSIOLOGICAL SOCIETY, 1996, Seiten 742-752, XP000882049
- VAN SUSANTE J.L.C., ET AL: "Culture of chondrocytes in alginate and collagen carriers gels" ACTA ORTHOP SCAND, XP000881912

## Beschreibung

Die Erfindung betrifft einen Träger für Zellkulturen, insbesondere einen Mikroträger für Bioreaktoren oder künstliche Organe, auf dessen Oberfläche trägergebundene Zellen angesiedelt sind.

Um die vorteilhafte Handhabung von Zellen in biotechnologischen Reaktoren zu ermöglichen sowie künstliche Organe mit lebenden Zellen herzustellen, ist es bekannt, Zellkulturen auf der Oberfläche eines Trägers anzusiedeln. Auf diese Weise lassen sich beispielsweise menschliche Zellen oder andere Säugetierzellen ortsfest fixieren, so daß sie in eine Nährlösung einbringbar sind und sich gegebenenfalls wieder mit dem Träger entnehmen lassen, ohne daß das Problem einer nachfolgenden Abscheidung zur Rückgewinnung aus der Lösung besteht. Während Zellen zur Herstellung von künstlichen Organen vorzugsweise auf einem Gerüst aus bioresorbierbarem Kunststoff angesiedelt sind, das entsprechend dem einzubringenden Implantat geformt ist, werden bei biotechnologischen Reaktoren meist lose Streuungen von Kugeln eingesetzt, die in Nährlösungen suspendiert sind. Die Durchmesser der Träger liegen in der Regel im Millimeter- oder Submillimeterbereich, so daß sie somit i. a. als Mikroträger zu bezeichnen sind. Als Materialien sind eine Vielzahl unterschiedlicher Werkstoffe gebräuchlich, beispielsweise Dextran, Polystyrol, Polyacrylamid, Zellulose, Gelatine oder Chitosan.

Die vorgenannten Materialien haben den Nachteil, dass sie in der Regel eine ungenügende Biokompatibilität aufweisen und daher insbesondere nicht in lebenden Geweben als Organersatz verwendbar sind.

Diese Vorgehensweise wird z. B. zur Behandlung der Diabetes mellitus vorgeschlagen, indem Mikroträger mit insulinproduzierenden Zellen in die Leber oder den Pankreas appliziert werden. Weiterhin ist bei gebräuchlichen Materialien meist eine erhebliche Begrenzung der möglichen Trägergrößen und
-geometrien gegeben. Schließlich dienen Bioreaktoren nicht nur der Produktion bestimmter, chemischer Substanzen sondern vielfach auch zur Vermehrung von Zellen. In diesem Fall ist es erforderlich, die Zellen nach Abschluss von Vermehrung und Wachstum von den Trägem abzulösen. Die zu diesem Zweck gebräuchlichen Enzyme bewirken häufig Schädigungen der zu gewinnenden Zellen, so dass ihre Verwendung problematisch ist (siehe hierzu McKeehan, W.L.; The effect of temperature during trypsin treatment on viability and multiplication potential of single normal human and chicken fibroblasts. 1977. Cell Biol. Int. Rep. 1; P. 335-343 und Freshney, R. I.; Culture of animals cells (4^{th} ed.) 2000. John Wiley & Sons, Inc., Publication. P. 160-161).

Weiterhin ist es im Stande der Technik bekannt, Alginate zur räumlichen Festlegung von Zellen zu verwenden. Aus Alginaten lassen sich auf einfache Weise Partikel unterschiedlicher Größe und Gestalt herstellen. Dabei ermöglicht es die Variation der Prozessparameter, beispielsweise der Alginatkonzentration, des Molekulargewichtes oder des Verhältnisses von Mannuron- zu Guluronsäure im Äusgangsstoff, Materialien mit unterschiedlichen Eigenschaften, etwa Partikel- oder Porengrößen, zu erzeugen. Ein weiterer Vorteil reiner Alginate ist ihre hervorragende Biokompatibilität. Zur Arisiedlung trägergebundener Zellen auf ihren äußeren Oberflächen sind Alginate jedoch nicht geeignet, weil sich die Zellen dort nicht festsetzen. Daher erfolgt die Fixierung der Zellen durch Einkapselung im Inneren von Alginatpartikeln, vorzugsweise in größeren Hohlräumen.

Vor diesem Hintergrund hat es sich die Erfindung zur Aufgabe gestellt, einen stabilen, biokompatiblen Träger für Zellkulturen zu entwickeln, auf dessen äußerer Oberfläche Zellen angesiedelt sind und von der sie sich schädigungsfrei ohne Enzyme ablösen lassen.

Diese Aufgabe wird erfindungsgemäß dadurch gelöst, daß der Träger aus einem Alginat besteht, die Oberfläche des Trägers mit einer Schicht aus einem Adhäsionsprotein überzogen ist und die Zellen auf dem Adhäsionsprotein befindlich sind.

Die zentrale Idee der Erfindung besteht darin, als Trägermaterial für die Zellkulturen ein Alginat zu verwenden, wobei sich Alginate mit hohen Mannuronsäureanteilen im Bereich von etwa 70 Prozent als vorteilhaft erwiesen haben. Um die Ansiedlung von Zellen auf der Oberfläche des Trägers zu ermöglichen, ist sie mit einer Schicht aus einem Adhäsionsprotein überzogen, auf dem sich die Zellen festsetzen. Somit sind die Zellen auf dem Adhäsionsprotein fixiert, das seinerseits am Alginat haftet.

Auf diese Weise entsteht ein Träger für Zellkulturen, der sich entsprechend den Eigenschaften des Alginats durch eine hohe Biokompatibilität auszeichnet. Er ist daher insbesondere für die in vivo-Geweberekonstruktion geeignet, zeigt aber auch eine gute chemische Stabilität in vitro. Unter Verwendung körpereigener Zellen lassen sich Abstoßungsreaktionen des Implantats vermeiden. Im Gegensatz zu bioresorbierbaren Materialien treten im Fall des vorgeschlagenen Trägers nach der Implantation auch keine Ausscheidungsprozesse von Abbauprodukten aus dem Körper auf, die in der Regel mit Entzündungen einhergehen. Weiterhin weist der Träger eine hohe mechanische Stabilität auf, da die dreidimensionale Struktur des Alginats durch das Adhäsionsprotein verstärkt und gestützt ist. Das Protein bewirkt eine sichere Haftung der auf ihm siedelnden Zellen, die somit ortsfest auf der Trägeroberfläche fixiert sind. Schließlich lassen sich empfindliche Zellen auch ohne Enzyme vom Trägermaterial ablösen, indem der Alginatanteil des Trägers - wie im folgenden noch näher erläutert - verflüssigt wird.

In einer vorteilhaften Weiterbildung der Erfindung findet als Adhäsionsprotein Kollagen Verwendung, das auf der Alginatoberfläche eine im wesentlichen zweidimensionale Faserstruktur ausbildet. Auf diese Weise ist eine sichere Haftung der Zellen auf dem Träger und dessen hohe mechanische Stabilität gewährleistet.

Für einen verbesserten Schutz der Adhäsionsproteine auf der Alginatoberfläche vor biologischen Abbauprozessen kann der Träger mit einer Lösung mit einem darin befindlichen chemischen Stabilisator behandelt werden. Hierfür werden insbesondere solche Substanzen vorgeschlagen, die zu einer Vernetzung der Adhäsionsproteine untereinander führen, speziell Tannin, Glutardialdehyd oder Carbodiimide.

Für den Alginatanteil des Trägers haben sich insbesondere Bariumalginate bewährt. Die Bariumionen, welche die Alginatmoleküle untereinander verbinden, bewirken eine hohe chemische Stabilität des Alginatgels sowohl unter in vitro- als in vivo-Bedingungen. Speziell die Beständigkeit von Calciumalginaten wird deutllich übertroffen.

Alginatpartikel zeigen erhebliche Größenvariationen in Abhängigkeit von Änderungen der umgebenden lonenkonzentration, insbesondere des pH-Wertes. Derartige Schwankungen können beispielsweise durch das Zellwachstum auf der Trägeroberfläche hervorgerufen werden. Um damit einhergehende Größenvariationen zu vermeiden, die zu einer Ablösung der Adhäsionsproteine von der Trägeroberfläche führen kann, wird vorgeschlagen, dem Alginat eine chemische Puffersubstanz zuzusetzen. Zu diesem Zweck ist u. a. der Wirkstoff RPMI 1640 geeignet.

Vorzugsweise umschließt das Alginat einen oder mehrere Hohlräume, deren Herstellung analog der im Stande der Technik bekannten Alginatkapseln vorgenommen wird (Dulieu, C., Poncelet D., Neufeld R.J.; Encapsulation and immobilization, in: Cell Encapsulation Technology and Therapeutics (Kühtreiber, W.M., Lanza, R.P., Chick, W.L., Eds.), pp. 3-17. Boston: Birkhäuser). In das Alginat oder darin befindliche Hohlräume sind Wirkstoffe einbringbar, mit denen sich das Zellwachstum beeinflussen lässt. Dabei kann es sich beispielsweise um Nährstoffe, Wachstumsfaktoren, Hormone oder Antibiotika handeln, die insbesondere das Wachstum junger Zellkulturen fördern, etwa in Konkurrenz zu unerwünschten Zellstämmen. Da die Bereitstellung der Wirkstoffe in unmittelbarer Umgebung der Zellen erfolgt, läßt sich eine ausreichende Konzentration auch bei geringer Menge sicherstellen, so daß erhebliche Kostenvorteile entstehen.

Einige Zellen gedeihen nur in Gegenwart anderer Zellsorten, mit denen Wechselbeziehungen bestehen. Unter verschiedenen Zellsorten sind dabei sowohl unterschiedliche Zellarten als auch Zellen der gleichen Art in verschiedenen Wachstumsstadien oder morphologischen Ausprägungen zu verstehen. Im allgemeinen sind Stoffwechselprodukte der einen Zellsorte für das Wachstum oder den Stoffwechsel der jeweils anderen Zellsorte erforderlich. Häufig läßt sich auch der Stoffumsatz einer Zelle durch Anwesenheit einer anderen Zellsorte erheblich steigern. Daher wird vorgeschlagen, auf dem Träger unterschiedliche, einander ergänzende Zellsorten anzusiedeln. Denkbar ist auch, anstelle der Einbringung von Wirkstoffen in den Träger solche Zellen auf oder in ihm anzusiedeln, welche den jeweiligen Wirkstoff produzieren.

Auch wenn mehrere Zellsorten auf einem Träger siedeln, ist häufig beabsichtigt, lediglich eine von ihnen oder ihre Stoffwechselprodukte zu nutzen, während die übrigen Zellsorten ausschließlich zur Wachstumsförderung oder Stoffwechselbeeinflussung dienen. Um die Gewinnung der vorgesehenen Zellsorte bzw. ihrer Stoffwechselprodukte zu erleichtern wird vorgeschlagen, daß auf der äußeren Oberfläche des Trägers und in seinem Inneren unterschiedliche Zellsorten vorhanden sind. Vorzugsweise befindet sich auf der äußeren Trägeroberfläche dabei eine Monokultur der primär genutzten Zellen. Der Stoffaustausch zwischen den Zellsorten kann aufgrund der Permeabilität auch durch das Alginat hindurch erfolgen.

Für den Einsatz in Bioreaktoren haben sich insbesondere kugelförmige Träger bewährt, die leicht herstellbar sind und eine gute mechanische Stabilität aufweisen.

Zur Herstellung von Implantaten wird der Träger dagegen vorzugsweise durch Abguß eines Gewebeabdrucks hergestellt. Somit lassen sich in den Abmessungen vorgegebene, dreidimensionale Implantate erzeugen, deren äußere Oberfläche in ihrer Struktur natürlichen Gewebematerialien entspricht. Im Inneren sind gegebenenfalls verschiedene Zellsorten in unterschiedlichen räumlichen Anordnungen vorhanden. Weiterhin ist es denkbar, Träger in größere Raumstrukturen, etwa ein Raumgitter zu integrieren. Auf diese Weise sind auch großflächige organdefekte oder -verluste ausgleichbar.

Zur Herstellung des vorgeschlagenen Trägers wird zunächst seine geometrische Gestalt aus dem Alginat geformt. Dabei wird eine Alginatlösung durch Zugabe einer gelierenden Substanz, etwa einer Mischung aus Bariumchlorid, Morpholinpropansulfonsäure und Kochsalz gehärtet. Um kugelförmige Träger zu erzeugen, wird die Alginatlösung zweckmäßig in ein Fällungsbad mit dem gelösten Geliermittel eingetropft, wobei sich zur Herstellung von Mikroträgem geringen Durchmessers luftbeaufschlagte Sprühdüsen eignen. Alternativ ist die Gelierung in Gussformen möglich, um Gewebeabdrücke zu erhalten. Während des Herstellungsprozesses lassen sich auf im Stande der Technik bekannte Weise (so z. B. Kühtreiber, W.M., Lanza R.P., Chick, W.L. (1999), Cell Encapsulation Technology and Therapeutics. Boston: Birkhäuser) auch Hohlräume, Zellen oder Wirkstoffe in das Innere der Alginatpartikel einbringen. Die Abtrennung der Partikel aus dem Fällungsbad erfolgt vorzugsweise mit einer Zentrifuge. Anschließend werden die geformten Alginatpartikel in eine verdünnte Lösung eines Adhäsionsproteins, vorzugsweise Kollagen eingebracht. Die Gelierung des Proteins bzw. Kollagens wird bewirkt, indem der pH-Wert der Lösung verändert wird. Dabei scheidet sich ein großer Teil auf der Trägeroberfläche ab, während überschüssige Anteile gelierten Proteins im Lösungsmittel verbleiben und sich abgießen lassen. Die Abtrennung der Träger aus der Lösung erfolgt vorzugsweise gleichfalls unter Verwendung einer Zentrifuge.

Für einen verbesserten Schutz der Adhäsionsproteine auf der Alginatoberfläche vor biologischen Abbauprozessen können die Träger anschließend in eine Lösung mit einem darin befindlichen chemischen Proteinstabilisator eingebracht werden, vorzugsweise Tannin in einer Konzentration von ca. 1 %. Nach einer Reaktionszeit, die im Fall der Tanninlösung bevorzugt ca. 10 Minuten beträgt, erfolgt die Abtrennung der Träger, zweckmäßig mit einer Zentrifuge.

Abschließend werden die Träger in eine Suspension vereinzelter, äufzubringender Zellen eingebracht, die sich selbstständig auf seiner Oberfläche ansiedeln. Die Vereinzelung der Zellen kann beispielsweise auf übliche Weise aus einem Zellverband oder von einer anderen Trägeroberfläche unter Verwendung des Enzyms Trypsin erfolgen.

Mit dem Ziel, Größenänderungen bei Schwankungen der lonenkonzentration mitder nachfolgenden Gefahr einer Ablösung des Adhäsionsproteins zu vermeiden, enthält der Träger zweckmäßig eine chemische Puffersubstanz, beispielsweise RPMI 1640. Zur Beaufschlagung mit der Puffersubstanz wird der aus Alginat bestehende Trägerkem vorzugsweise in einer Lösung der Puffersubstanz gelagert, wobei zweckmäßige Zeiträume abhängig von den Abmessungen des Trägers zwischen einigen Stunden und Tagen liegen. Anschließend erfolgt auf vorbeschriebene Weise das Aufbringen der Proteinschicht und Zellen auf den Träger.

Zur Gewinnung der Zellen von der Oberfläche lässt sich der erfindungsgemäße Träger, wie aus Lindl, T.; Zell- und Gewebekultur (4te Auflage), 2000; Spektrum Akademischer Verlag, S. 99, bekannt, in eine Lösung lytischer Enzyme, etwa Trypsin, einbringen. Auf diese Weise wird die Zellablösung von der Oberfläche bewirkt. Alternativ besteht die Möglichkeit seines Einbringens in eine Lösung mit darin befindlichem Komplexbildner, vorzugsweise EDTA (Ethylendiamintetraessigsäure). Die EDTA-Konzentration liegt bevorzugt um 5 Millimol, wobei eine calcium- und magnesiumionenfreie, phosphatgepufferte Kochsalzlösung ein geeignetes Lösungsmittel darstellt. Die EDTA-Lösung bewirkt eine Auflösung des Alginats, wobei die Prozesstemperatur zweckmäßig etwa 35 bis 40 Grad Celsius ist. Die geeignete Zeitdauer der Einwirkung beträgt abhängig von geometrischen Parametern des Trägers zwischen einer und einigen zehn Minuten. Auf diese Weise erhält man freie Zellschichten auf dem Adhäsionsproteinsubstrat, die sich aus der Lösung abtrennen lassen. Somit ist eine rasche Gewinnung von Zellen und Zellgemischen ohne Schädigung durch lytische Enzyme möglich. Denkbar ist, die nach Entfernung des Alginats verbleibende Hohlkugel aus Adhäsionsprotein mit den Zellen zunächst in einer Nährlösung zum weiteren Zellwachstum zu belassen, wobei sich das Wachstum von Zellen im Inneren der Proteinhülle aufgrund der verbesserten Diffusion erheblich beschleunigt.

Um auch die Adhäsionsproteinschicht des Trägers aufzulösen, wird er oder die von ihm abgetrennten Zellen mit der anhaftenden Proteinschicht in eine Lösung eines auf das Protein lytisch wirkenden Enzyms eingebracht, beispielsweise Kollagenase im Fall von Kollagen. Abhängig von der Zielsetzung kann der Einsatz des Enzyms in unterschiedlichen Verfahrensschritten erfolgen. Soll lediglich eine vollständige Auflösung des Trägers vorgenommen werden, kann das Enzym der EDTA-Lösung zugesetzt werden oder nach Abtrennen der Zellen aus der Lösung auf sie einwirken. Um dagegen Zellen im Inneren des Alginats vor nachteiligen Einflüssen des Enzyms zu schützen, erfolgt die Auflösung der Proteinschicht vor dem Einbringen des Trägers in die EDTA-Lösung. Somit wird ein unmittelbarer Kontakt der Zellen im Alginatinneren mit dem Enzym vermieden.

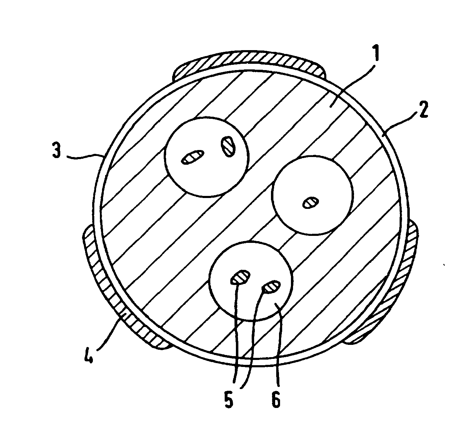

Weitere Einzelheiten, Merkmale und Vorteile der Erfindung lassen sich dem nachfolgenden Beschreibungsteil entnehmen, in dem ein Ausführungsbeispiel anhand der Zeichnung näher erläutert ist. Sie zeigt den Querschnitt durch einen erfindungsgemäßen Träger für Zellkulturen in prinzipienhafter Darstellung.

Der Kern des Trägers besteht aus Alginat (1), dem bevorzugt ein Ionenpuffer sowie gegebenenfalls Wirkstoffe zur Beeinflussung des Zellwachstums zugesetzt sind. Seine Oberfläche ist mit einer durchgehenden Schicht eines Adhäsionsproteins (2), beispielsweise Kollagen, überzogen. Auf der Oberfläche (3) des Adhäsionsproteins (2) haften Zellen (4), die vermehrt werden sollen oder gewünschte Wirkstoffe produzieren. Dagegen zeigen Untersuchungen, daß auf einer proteinfreien Alginatoberfläche keine Ansiedlung von Zellen (4) oder Zellverbänden erfolgt.

Häufig ist es erforderlich, verschiedene Zellen (4, 5) in Co-Kultur zu züchten, wobei die unterschiedlichen Zellsorten beispielsweise in einer Stoffwechselbeziehung miteinander stehen. Ist lediglich die Gewinnung einer Sorte der Zellen (4, 5) beabsichtigt, so bietet sich eine räumliche Trennung an. Zu diesem Zweck ist der Träger in seinem Inneren mit Hohlräumen (6) versehen, in denen die Zellen (5) eingeschlossen sind. Somit lassen sich beispielsweise die Zellen (4) sortenrein von der Oberfläche (3) gewinnen, indem das Adhäsionsprotein (2) durch eine Protease aufgelöst wird, welche das Alginat (1) unbeeinflußt läßt. Nach erneuter Aufbringung eines Adhäsionsproteins (2) auf den Alginatkern sowie einzelner Zellen (4) auf die Oberfläche (3) kann ein weiterer Wachstumszyklus erfolgen. Das Alginat (1) mit den Zellen (5) bleibt dabei erhalten, so daß sich Prozeßaufwand und -kosten erheblich verringern.

Im Ergebnis entsteht somit ein stabiler, biokompatibler Träger für trägergebundene Zellen, der sich sowohl zur Herstellung künstlicher Organe als auch zum Einsatz in biotechnischen Reaktoren eignet und von dem sich Zellen alternativ mit einer Proteinase zur Auflösung des Adhäsionsproteins oder enzymfrei durch Auflösung des Alginats abtrennen lassen.

## Patentansprüche

1. Träger für Zellkulturen, insbesondere Mikroträger für Bioreaktoren oder künstliche Organe, auf dessen Oberfläche trägergebundene Zellen angesiedelt sind, **dadurch gekennzeichnet, daß**
- der Träger aus einem Alginat (1) besteht,
- die Oberfläche (3) des Trägers mit einer Schicht aus einem Adhäsionsprotein (2) überzogen ist
- und die Zellen (4) auf dem Adhäsionsprotein (2) befindlich sind.

2. Träger nach Anspruch 1, **dadurch gekennzeichnet, daß** das Adhäsionsprotein (2) Kollagen ist.

3. Träger nach Anspruch 1 oder 2, **dadurch gekennzeichnet, daß** das Adhäsionsprotein (2) chemisch stabilisiert ist.

4. Träger nach Anspruch 3, **dadurch gekennzeichnet, daß** der Stabilisator Tannin, Glutardialdehyd oder ein Carbodiimid ist.

5. Träger nach einem der vorhergehenden Ansprüche, **dadurch gekennzeichnet, daß** das Alginat (1) Bariumalginat ist.

6. Träger nach einem der vorhergehenden Ansprüche, **dadurch gekennzeichnet, daß** das Alginat (1) eine chemische Puffersubstanz enthält.

7. Träger nach einem der vorhergehenden Ansprüche, **dadurch gekennzeichnet, daß** das Alginat (1) einen oder mehrere Hohlräume (6) umschließt.

8. Träger nach einem der vorhergehenden Ansprüche, **dadurch gekennzeichnet, daß** das Alginat (1) oder die Hohlräume (6) Zellen oder Wirkstoffe zur Beeinflussung des Zellwachstums enthalten.

9. Träger nach einem der vorhergehenden Ansprüche, **dadurch gekennzeichnet, daß** unterschiedliche Sorten von Zellen (4, 5) auf dem Träger angesiedelt sind.

10. Träger nach Anspruch 9, **dadurch gekennzeichnet, daß** die Sorten auf der Oberfläche (3) und in den Hohlräumen (6) befindlicher Zellen (4, 5) unterschiedlich sind.

11. Träger nach einem der vorhergehenden Ansprüche, **gekennzeichnet durch** eine kugelförmige Gestalt.

12. Träger nach einem der Ansprüche 1 bis 10, **dadurch gekennzeichnet, daß** der Träger die Gestalt eines biologischen Gewebes aufweist.

13. Träger nach einem der vorhergehenden Ansprüche, **dadurch gekennzeichnet, daß** der Träger in eine Raumstruktur integriert ist.

14. Verfahren zur Herstellung von Trägern nach einem der vorhergehenden Ansprüche, **gekennzeichnet durch** folgende Verfahrensschritte:
- Formung der Träger aus Alginat (1),
- Einbringen der Träger in eine Lösung eines Adhäsionsproteins (2),
- Gelierung des Adhäsionsproteins (2),
- Abtrennung der Träger aus der Lösung,
- Einbringen der Träger in eine Suspension der aufzubringenden Zellen (4).

15. Verfahren nach Anspruch 14, **dadurch gekennzeichnet, daß** das Adhäsionsprotein (2) Kollagen ist.

16. Verfahren nach Anspruch 14 oder 15, **dadurch gekennzeichnet, daß** der Träger vor dem Einbringen in die Kollagenlösung in einer chemischen Puffersubstanz gelagert wird.

17. Verfahren nach einem der Ansprüche 14 bis 16, **dadurch gekennzeichnet, daß** die Träger nach dem Abtrennen aus der Lösung des Adhäsionsproteins (2) in eine Lösung mit einer proteinstabilisierenden Substanz eingebracht und nachfolgend daraus abgetrennt werden.

18. Verfahren zum Ablösen der Zellen (4) von der Oberfläche (3) eines Trägers nach einem der Ansprüche 1 bis 13, **gekennzeichnet durch** folgende Verfahrensschritte:
- Einbringen des Trägers in eine Lösung mit einem Komplexbildner,
- Belassen des Trägers in der Lösung des Komplexbildners bis zur Auflösung des Alginats (1),
- Abtrennen der Zellen (4, 5) aus der Lösung.

19. Verfahren nach Anspruch 18, **dadurch gekennzeichnet, daß** der Komplexbildner EDTA ist.

20. Verfahren nach Anspruch 18 oder 19, **dadurch gekennzeichnet, daß** der Träger oder die abgetrennten Zellen (4, 5) in eine Lösung eines Enzyms eingebracht werden, welches das Adhäsionsprotein (2) löst.

## Claims

1. Substrate for cell cultures, in particular a micro-substrate for bioreactors or artificial organs, on the surface of which substrate-bound cells are attached, **characterised in that**
- the substrate consists of an alginate (1),
- the surface (3) of the substrate is covered with a layer of an adhesion protein (2)
- and the cells (4) are located on the adhesion protein (2).

2. Substrate according to claim 1, **characterised in that** the adhesion protein (2) is collagen.

3. Substrate according to claim 1 or 2, **characterised in that** the adhesion protein (2) is chemically stabilised.

4. Substrate according to claim 3, **characterised in that** the stabiliser is tannin, glutardialdehyde or a carbodiimide.

5. Substrate according to one of the preceding claims, **characterised in that** the alginate (1) is barium alginate.

6. Substrate according to one of the preceding claims, **characterised in that** the alginate (1) contains a chemical buffer substance.

7. Substrate according to one of the preceding claims, **characterised in that** the alginate (1) encloses one or more cavities (6).

8. Substrate according to one of the preceding claims, **characterised in that** the alginate (1) or the cavities (6) contain cells or active substances for influencing the cell growth.

9. Substrate according to one of the preceding claims, **characterised in that** different sorts of cells (4, 5) are attached to the substrate.

10. Substrate according to claim 9, **characterised in that** the sorts of cells (4, 5) on the surface (3) and in the cavities (6) are different.

11. Substrate according to one of the preceding claims, **characterised by** a spherical shape.

12. Substrate according to one of claims 1 to 10 **characterised in that** the substrate has the shape of a biological tissue.

13. Substrate according to one of the preceding claims, **characterised in that** the substrate is integrated into a three-dimensional structure.

14. Method for producing substrates according to one of the preceding claims, **characterised by** the following process steps:
- forming the substrate from alginate (1),
- introducing the substrate into a solution of an adhesion protein (2),
- gelling the adhesion protein (2),
- separation of the substrate from the solution,
- introduction of the substrate into a suspension of the cells (4) to be attached,

15. Method according to claim 14, **characterised in that** the adhesion protein (2) is collagen.

16. Method according to claim 14 or 15, **characterised in that** the substrate is immersed in a chemical buffer substance before introduction into the collagen solution.

17. Method according to one of claims 14 to 16, **characterised in that**, after separation from the solution of the adhesion protein (2), the substrates are introduced into a solution with a protein-stabilising substance and subsequently separated from it.

18. Method for detaching the cells (4) from the surface (3) of a substrate according to one of claims 1 to 13, **characterised by** the following process steps:
- Introduction of the substrate into a solution containing a complexing agent
- Leaving the substrate in the solution of the complexing agent until the alginate (1) dissolves
- Separation of the cells (4, 5) from the solution.

19. Method according to claim 18, **characterised in that** the complexing agent is EDTA.

20. Method according to claim 18 or 19, **characterised in that** the substrate or the separated cells (4, 5) are introduced into a solution of an enzyme, which dissolves the adhesion protein (2).

## Revendications

1. Support pour cultures cellulaires, notamment microsupport pour bioréacteurs ou organes artificiels sur la surface desquels sont implantées des cellules liées à des supports, **caractérisé par le fait que**
• le support est composé d'un alginate (1),
• la surface (3) du support est recouverte d'une couche de protéine d'adhésion (2)
• et les cellules (4) se trouvent sur la protéine d'adhésion (2).

2. Support selon la revendication 1, **caractérisé par le fait que** la protéine d'adhésion (2) est du collagène.

3. Support selon les revendications 1 ou 2, **caractérisé par le fait que** la protéine d'adhésion (2) est stabilisée chimiquement.

4. Support selon la revendication3, **caractérisé par le fait que** le stabilisateur est du tannin, du glutardialdéhyde ou un carbodiimide.

5. Support selon une des revendications précédentes **caractérisé par le fait que** l'alginate (1) est de l'alginate de barium.

6. Support selon une des revendications précédentes **caractérisé par le fait que** l'alginate (1) entoure une substance chimique tampon.

7. Support selon une des revendications précédentes **caractérisé par le fait que** l'alginate (1) renferme une ou plusieurs cavités (6).

8. Support selon une de revendications précédentes **caractérisé par le fait que** l'alginate (1) ou les cavités (6) contiennent des cellules ou des agents actifs destinés à influencer la croissance des cellules.

9. Support selon une des revendications précédentes **caractérisé par le fait que** différents types de cellules (4, 5) sont implantées sur le support.

10. Support selon la revendication 9, **caractérisé par le fait que** les types de cellules (4, 5) se trouvant sur la surface (3) et dans les cavités (6) sont différents.

11. Support selon une des revendications précédentes **caractérisé par** une forme sphérique.

12. Support selon une des revendications 1 à 10 **caractérisé par le fait que** le support présente la forme d'un tissu biologique.

13. Support selon une des revendications précédentes **caractérisé par le fait que** le support est intégré dans une structure spatiale.

14. Support selon une des revendications précédentes **caractérisé par** les étapes de procédé suivantes :
• formation des supports en alginate (1),
• apport des supports dans une solution de protéine d'adhésion (2),
• gélification de la protéine d'adhésion (2)
• séparation des supports de la solution,
• apport des supports dans une suspension des cellules devant être produites (4).

15. Support selon la revendication 14, **caractérisé par le fait que** la protéine d'adhésion (2) est du collagène.

16. Procédé selon la revendication 14 ou 15 **caractérisé par le fait que** le support est stocké avant son apport dans la solution de collagène dans une substance chimique tampon.

17. Procédé selon une des revendications 14 à 16 **caractérisé par le fait que** les supports sont mis, après leur séparation de la solution de la protéine d'adhésion (2) dans une solution avec une substance stabilisant les protéines et en sont ensuite séparées.

18. Procédé pour le détachement des cellules (14) de la surface (3) d'un support d'après une des revendications 1 à13, **caractérisé par** les étapes de procédé suivantes :
• Apport du support dans une solution avec un agent de complexation,
• Abandon du support dans la solution de l'agent de complexation jusqu'à la dissolution de l'alginate (1),
• séparation des cellules (4, 5) des cellules.

19. Procédé selon la revendication 18, **caractérisé par le fait que** l'agent de complexation est de l'EDTA.

20. Procédé selon la revendication 18 ou 19 **caractérisé par le fait que** le support ou les cellules séparées (4, 5) sont mises dans une solution d'un enzyme dissolvant la protéine d'adhésion (2).
